(19) Europäisches Patentamt
European Patent Office
Office européen des brevets

(11) Publication number: **0 351 928 B1**

(12) # EUROPEAN PATENT SPECIFICATION

(45) Date of publication of patent specification: **09.06.93**

(51) Int. Cl.5: **C07C 303/06**, C07C 309/08, C07C 309/20

(21) Application number: **89201907.6**

(22) Date of filing: **19.07.89**

(54) **A process for the preparation of internal olefin sulphonates.**

(30) Priority: **20.07.88 GB 8817293**

(43) Date of publication of application:
**24.01.90 Bulletin 90/04**

(45) Publication of the grant of the patent:
**09.06.93 Bulletin 93/23**

(84) Designated Contracting States:
**BE DE ES FR GB IT NL SE**

(56) References cited:
**US-A- 3 424 694**
**US-A- 4 183 867**
**US-A- 4 248 793**

**TENSIDE DETERGENTS, vol. 22, no. 4, July/August 1985, pages 193-195, Carl Hanser Verlag, München, DE; D.W. ROBERTS et al.: "Why internal olefins are difficult to sulphonate"**

(73) Proprietor: **SHELL INTERNATIONALE RESEARCH MAATSCHAPPIJ B.V.**
**Carel van Bylandtlaan 30**
**NL-2596 HR Den Haag(NL)**

(72) Inventor: **Stapersma, Johan**
**Badhuisweg 3**
**NL-1031 CM Amsterdam(NL)**
Inventor: **Van Ginkel, Roelof**
**Badhuisweg 3**
**NL-1031 CM Amsterdam(NL)**

EP 0 351 928 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The invention relates to a process for the preparation of internal olefin sulphonates and to internal olefin sulphonates as novel products.

It is generally known that internal olefins are more difficult to sulfonate than alpha-olefins (see "Tenside Detergents" 22 (1985) 4, pp 193-195). In the article, entitled "Why internal olefins are difficult to sulphonate", the authors state that by the sulfonation of various commercial and laboratory-produced internal olefins, using falling film reactors, internal olefins gave conversions of below 90% and further they state that it was found necessary to raise the $SO_3$ : internal olefin mol ratio to over 1.6:1 in order to achieve conversions above 95%; furthermore the resulting products were very dark in colour and had high levels of di- and polysulphonated products.

In the US Patent Specifications 4,183,867 and 4,248,793 are disclosed processes, carried out in a falling film reactor, for the preparation of light-colored internal olefin sulphonates, however the amounts of unreacted internal olefins are still between 10 and 20 per cent and at least 20 per cent respectively in the disclosed processes and special measures must be taken to remove the unreacted internal olefins. The internal olefin sulphonates containing between 10 and 20 per cent and at least 20 per cent of unreacted internal olefins (also called free oil) must be purified before being used. Consequently the preparation of internal olefin sulphonates having the desired light colour and with the desired low free oil content offers substantial difficulties.

It is an object of the invention to prepare internal olefin sulphonates with a low free oil content, with a light colour and avoiding the purification step.

Applicant has now found a process for the preparation of internal olefin sulphonates which comprises reacting in a film reactor an internal olefin having from 8 to 26 carbon atoms with a sulphonating agent, in a mol ratio of sulphonating agent to internal olefin of 1:1 to 1.25:1 while cooling the reactor with a cooling means having a temperature not exceeding 35 °C, and directly neutralizing the obtained reaction product of the sulphonating step and, without extracting the unreacted internal olefin, hydrolyzing the neutralized reaction product.

The invention furthermore relates to the internal olefin sulphonates having from 8 to 26 carbon atoms characterized by containing at least 10% by weight of beta-hydroxy sulphonates, preferably at least 25% by weight of beta-hydroxy sulphonates, more preferably at least 50% by weight of beta-hydroxy sulphonates. Especially preferred are internal olefin sulphonates having from 50 to 90% by weight of beta-hydroxy sulphonates, even more preferred are internal olefin sulphonates having from 60 to 85% by weight of beta-hydroxy sulphonates.

In the preparation of sulphonates derived from internal olefins, the internal olefins are reacted with a sulphonating agent, preferably sulphur trioxide, with the formation of beta-sultones and some alkene sulphonic acids.

The reaction products are neutralized and hydrolyzed. Under certain circumstances, for instance ageing the beta-sultones are converted into gamma-sultones, which on their turn may be converted into delta-sultones. After neutralization and hydrolysis the gamma-hydroxy sulphonates and delta-hydroxy sulphonates are obtained respectively. A disadvantage of the gamma-and delta-sultones is that they are more difficult to hydrolyze than beta-sultones.

An advantage of the beta-sultones is that hydrolysis proceeds easier than with gamma- and delta-sultones.

The beta-sultones, after hydrolysis, give beta-hydroxy sulphonates. The internal olefin sulphonates containing more then 10% by weight of beta-hydroxy sulphonates are novel products.

The internal olefins used as starting material in the present invention are those having 8 to 26 carbon atoms, preferably 13 to 22 carbon atoms. Examples of the internal olefins are those having the general formula

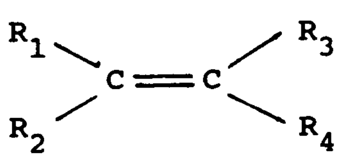

wherein each of the groups $R_1$, $R_2$, $R_3$ and $R_4$ independently are linear or branched alkyl groups or hydrogen and the total number of carbon atoms of $R_1$, $R_2$, $R_3$ and $R_4$ is from 6 to 24, with the proviso that at least one of $R_1$ and $R_2$ and one of $R_3$ and $R_4$ is an alkyl group.

The sulphonation of the internal olefins is preferably carried out with sulphur trioxide in a film reactor. Preferred is the falling film reactor.

The cooling means, which is preferably water, has a temperature not exceeding 35 °C, especially a temperature in the range of from 0 °C to 25 °C. Depending upon the circumstances lower temperatures may be used as well.

In the process according to the invention it is preferred that the reaction mixture from the film reactor is not aged during a long time. The reaction mixture may be aged during a short period, but better is to age not at all, since then the highest percentage of beta-hydroxy sulphonates may be obtained. For that purpose the reaction mixture is led to a neutralization hydrolysis unit.

The neutralization/hydrolysis is carried out with a water soluble base, such as sodium hydroxide or sodium carbonate. Also the corresponding bases derived from potassium or ammonium are suitable. The neutralization of the reaction product from the falling film reactor is generally carried out with excess of base, calculated on the acid component. Generally neutralization is carried out at a temperature in the range of from 0 °C to 80 °C.

Hydrolysis may be carried out at a temperature in the range of from 100 °C to 250 °C, preferably 130 °C to 200 °C. The hydrolysis time generally may be from 5 min to 4 hours. Alkaline hydrolysis may be carried out with hydroxides, carbonates or bicarbonates of (earth) alkali metals.

As has already been explained above the beta-sultone of an internal olefin is converted in the process according to the invention into beta-hydroxy sulphonate according to the equation.

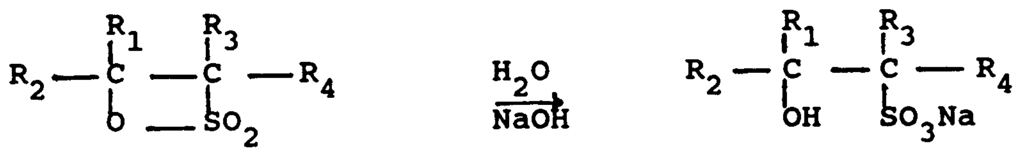


wherein $R_1$, $R_2$, $R_3$ and $R_4$ have the above significance. It has been found that the amount of beta-hydroxy sulphonate is dependent on the amount of beta-sultone. Under the rather mild reaction circumstances the beta-sultones are readily formed and since the slow-ageing step is generally avoided, gamma- and delta-sultones are formed only to a minor extent.

The process according to our invention may be carried out batchwise, semi-continuously or continuously. The reaction is generally performed in a falling film reactor which is cooled by flowing a cooling means at the outside walls of the reactor. At the inner walls of the reactor the internal olefin flows in downward direction. $SO_3$ is diluted with a stream of nitrogen, air or any other inert gas into the reactor. The concentration of $SO_3$ generally lies between 2 and 4 per cent by volume on the volume of the carrier gas.

In the preparation of internal olefin sulphonates, derived from olefins with more than 14 carbon atoms, it is required that in the neutralization/hydrolysis very intimate mixing of the reactor product and the aqueous base is achieved. This can be done by e.g. efficient stirring, the addition of a polar cosolvent (lower alcohol) or a phase-transfer agent.

The general analytical procedures are given in the footnotes of the Table. More details of the procedures used can be found in "Technical Bulletin Shell Chemical Company" SC-181-77, entitled "Alpha olefin sulphonates analysis procedures", May 1976.

The following examples illustrate the invention.

EXAMPLE 1-7

The sulphonation of a mixture of $C_{13-14}$-internal olefins was carried out in a stainless steel reactor, having a diameter of 0.9 cm and a length of 2 m.

The sulphur trioxide was prepared by reacting sulphur dioxide with oxygen (dry air) over a vanadium pentoxide catalyst at about 450 °C. The reactor was cooled by flowing water of low temperature along the outside of the stainless steel reactor tube.

The $C_{13-14}$-internal olefins flowed along the inner part of the reactor walls as a flowing film in downward direction and they reacted with the sulphur trioxide. The reaction product was subsequently conducted to a stainless steel autoclave, equipped with a magnetical stirrer, and which autoclave had been charged with an aqueous sodium hydroxide solution. The mixture was well stirred in the autoclave. The autoclave was then closed and heated to the desired temperature and kept at that temperature for the desired period of time. Subsequently the autoclave was cooled to ambient temperature and the contents

were analyzed by appropriate means.

The cooling water, cooling the reactor, had an inlet temperature of 8 °C and an outlet temperature of 13 °C, flowing countercurrently with the feed/$SO_3$ stream.

Hydrolysis took place during 1 h at 160 °C with 20% molar excess of NaOH (calculated on product as out of the reactor) to obtain active material in an almost quantitative yield.

In example 5 the cooling water had an inlet temperature of 10 °C and an outlet temperature of 15 °C, while in examples 6 and 7 the inlet temperature was 12 °C and the outlet temperature 17 °C. In example 5 hydrolysis took place at 140 °C during 1 h.

The free oil content, the $Na_2SO_4$ content and the residual sultones content are calculated on amount of active material. In example 7 after the sulphonation, but before neutralization/hydrolysis, the product was aged during 30 min at 30 °C.

In the following Table the results are shown.

TABLE

| Ex | feed rate (mol/h) | $SO_3$/feed mol/mol | $SO_3$ vol% in gas | free oil[1)] (%wt) | $Na_2SO_4$[2)] (%wt) | Klett colour[3)]/ colour bleached[6)] | beta-hydroxy sulphonates[4)] (%mol) | residual sultones[5)] (ppm) |
|---|---|---|---|---|---|---|---|---|
| 1 | 10 | 1.01 | 2.3 | 5.3 | 3.1 | 260/115 | | |
| 2 | 10 | 1.11 | 2.3 | 3.8 | 3.2 | 270/120 | 75 | |
| 3 | 10 | 1.07 | 2.3 | 4.6 | 3.2 | 350/145 | 80 | |
| 4 | 15 | 1.19 | 3.5 | 5.4 | 2.0 | 550/230 | | |
| 5 | 10 | 1.12 | 2.3 | 2.2 | 3.2 | 250/--- | 75 | < 20 |
| 6 | 10 | 1.12 | 2.3 | 2.8 | 3.0 | 280/--- | 85 | 25 |
| 7 | 10 | 1.12 | 2.3 | 4.2 | 3.3 | 520/--- | 60 | 380 |

1) free oil is the petroleum ether extractable material in an aqueous-alcoholic solution of internal olefin sulphonates

2) determined according to SMS 1721-76

3) Klett-Summerson photoelectric colorimeter, equipped with a KSNo42 color filter, and a 2x4 cm glass cell, measured on a 5% active material solution

4) beta-hydroxy sulphonate determined by $^{13}C$ NMR spectroscopy

5) gas chromatography of the free oil using $C_{16}$-terminal delta-sultone as internal standard

6) bleached with 1% by weight of hydrogen peroxide, calculated on weight of active material

EXAMPLE 8

The sulphonation of $C_{18}$-internal olefin was performed in a glass reactor, having a diameter of 0.5 cm and a length of 1 m.

The sulphonation reaction was performed in the same way as described in the previous examples under the following conditions:

| | |
|---|---|
| cooling water temperature | 15 °C |
| $SO_3$/feed ratio, mol/mol | 1,2 |
| $SO_3$ in $N_2$, % vol. | 3 |
| feed rate, mol/h | 1.5 |
| neutralization, NaOH on acid product, mol/mol | 1.4 |
| hydrolysis time, h, | 1 |
| hydrolysis temp., °C, | 160 |

The neutralization and hydrolysis were carried out with NaOH in a water/ethanol solution (vol/vol, 75:25). The product contained 1.6% by weight of free oil and 2.4% by weight of $Na_2SO_4$ (calculated on amount of active material). 80% by mol of the product was identified as beta-hydroxy sulphonate.

EXAMPLE 9

The sulphonation of $C_{18}$-internal olefin was carried out under the same conditions as disclosed in example 8. The neutralization and hydrolysis (1h at 160 °C) were carried out with an aqueous sodium hydroxide solution in the presence of an amount of $C_{13-14}$-internal olefin sulphonate (10% by weight on acid intake).

The product contained 7.9% by weight of free oil and 3.9% by weight of $Na_2SO_4$. The product had a colour index of 200 (according to Klett).

EXAMPLE 10

The sulphonation of $C_{18}$-internal olefin was carried out under the same conditions as disclosed in example 8. The neutralization and hydrolysis (1 h at 160 °C) were carried out with an aqueous sodium hydroxide solution under vigorous stirring of the obtained mixture.

The product contained 7.4% by weight of free oil and 5.4% by weight of $Na_2SO_4$. The product had a colour index of 140 (according to Klett).

EXAMPLE 11

The sulphonation of $C_{18}$-internal olefin was carried out under the same conditions as disclosed in example 8. The neutralization and hydrolysis (1 h at 160 °C) were carried out with an aqueous sodium hydroxide solution in the presence of an amount of DOBANOL 91-10 (5% by weight on acid intake). DOBANOL 91-10 is an alcohol mixture of primary, linear $C_9$, $C_{10}$, and $C_{11}$ alcohols (weight ratio: 18/50/32) that is ethoxylated to an averaged ethylene oxide number of 10. The product contained 5.5% by weight of free oil and 4.8% by weight of $Na_2SO_4$.

EXAMPLE 12

The sulphonation of $C_{18}$-internal olefin was carried out under the same conditions as disclosed in example 8. The neutralization and hydrolysis (1 h at 160 °C) were carried out with an aqueous sodium hydroxide solution under vigorous stirring and, at the same time, in the presence of an amount of DOBANOL 91-10 (5% by weight on acid intake).

The product contained 3.1% by weight of free oil and 4.6% by weight of $Na_2SO_4$.

EXAMPLE 13

The sulphonation of $C_{13-14}$-internal olefins was carried out in the reactor as described in example 8. The sulphonation reaction was performed under the following conditions:

| | |
|---|---|
| cooling water temperature, °C | 15 |
| $SO_3$/feed ratio, mol/mol | 1.1 |
| $SO_3$ in $N_2$, % vol. | 2.8 |
| feed rate, mol/h | 1.5 |
| neutralization, NaOH on acid product, mol/mol | 1.2 |
| hydrolysis time, h | 1 |
| hydrolysis temp., °C | 160 |

The product contained 2.1% by weight of free oil and 2.4% by weight of $Na_2SO_4$. The product (not bleached) had a colour index of 90 (according to Klett).

EXAMPLE 14

Sulphonation

The sulphonation of an internal olefin mixture containing internal olefins in the $C_{15}$-$C_{19}$ range ($\leqq C_{14}$ 2%; $C_{15}$ 25%; $C_{16}$ 26%; $C_{17}$ 24%; $C_{18}$ 18%; $\geqq C_{20}$ 5% by weight; purity: ca. 98%; remaining 2%: paraffins) was carried out under the same sulphonation conditions as disclosed in example 8.

Continuous neutralization

The acid product mixture from the falling film sulphonation reactor was directly pumped continuously into a continuously stirred tank reactor (CSTR) near and at the level of the 6-(flat-)bladed impeller (diameter: 35 mm). At the same time aqueous caustic (1.2 eg as calculated on acid intake) was also continuously pumped into the CSTR near and at the same level of the impeller. The volume of the CSTR was kept at 500 ml by siphoning off the product at the top of the reactor. Operated in this way the average residence time in the CSTR amounted ca. 20 min. The CSTR was maintained at 30 °C by external cooling/heating whilst the impeller speed was 750 rotations per minute, 1000 rotations per minute and 1500 rotations per minute respectively; in the set-up described the power input due to stirring amounted to ca. 1.2-9.7 kW/$m^3$. In this range the analytical data of the products obtained were independent of the power input.

The continuous neutralization was started up with the CSTR filled with $C_{15-19}$ internal olefin sulphonates with the following analytical data

active matter, AM (%w) : 26-31
free oil (%w on AM) : 6-10
inorganic sulphate (%w on AM) : 3-8

that was obtained from experiments without an aging step between the sulphonation and neutralization step, and containing 70-90% of beta-hydroxysulphonates. By starting up and operating the neutralization reactor continuously in this way, the product formed itself acts as an in situ generated emulsifier.

Hydrolysis

After the start of the continuous neutralization, samples were taken from the product stream only after waiting for at least 1 hour (i.e. after three residence times) and such samples were hydrolyzed batch-wise for 1 hour at 160 °C.

The product contained 6.7% by weight of free oil (of this 6.7%, however, ca. 2% is paraffins; therefore, the actual free oil content calculated on 100% olefin is 4.7% by weight) and 4.9% by weight of $Na_2SO_4$, calculated on active matter basis as indicated above. The product (not bleached) had a colour index of 500 (according to Klett). Approx. 85% by mol of the product was identified as beta-hydroxysulphonate.

## Claims

**1.** A process for the preparation of internal olefin sulphonates which comprises reacting in a film reactor an internal olefin having from 8 to 26 carbon atoms with a sulphonating agent, in a mol ratio of sulphonating agent to internal olefin of 1:1 to 1.25:1 while cooling the reactor with a cooling means having a temperature not exceeding 35 °C, and directly neutralizing the obtained reaction product of the sulphonating step and, without extracting the unreacted internal olefin, hydrolyzing the neutralized reaction product.

2. A process as claimed in claim 1, wherein the cooling means is water having a temperature in the range of from 0 °C to 25 °C.

3. A process as claimed in claims 1-2, wherein the sulphonating agent is sulphur trioxide.

4. A process as claimed in one or more of the claims 1-3, wherein the internal olefin comprises from 13 to 22 carbon atoms.

5. A process as claimed in one or more of the claims 1-4, wherein the process is carried out in such a manner that an ageing step is omitted at all.

6. A process as claimed in one or more of the claims 1-5, wherein the reaction is performed in a falling film reactor.

7. A process as claimed in one or more of the claims 1-6, wherein during neutralization/hydrolysis the reaction product of the sulphonating step is intimately mixed with an aqueous base solution.

8. A process as claimed in claim 7 wherein the mixing is effected by stirring, by the addition of a co-solvent or by the addition of a phase-transfer agent.

9. Internal olefin sulphonates having from 8 to 26 carbon atoms characterized in that these internal olefin sulphonates comprise at least 10% by wt of beta-hydroxy sulphonates, on the weight of the total sulphonates.

10. Internal olefin sulphonates as claimed in claim 9, characterized by at least 25% by wt of beta-hydroxy sulphonates.

11. Internal olefin sulphonates as claimed in claim 10, characterized by at least 50% by wt of beta-hydroxy sulphonates.

12. Internal olefin sulphonates as claimed in claim 11, characterized by 50 to 90% by wt of beta-hydroxy sulphonates.

13. Internal olefin sulphonates as claimed in claim 12, characterized by 60 to 85% by wt of beta-hydroxy sulphonates.

14. Internal olefin sulphonates as claimed in one or more of the claims 9-13, characterized in that these internal olefin sulphonates contain an amount of unsaturated sulphonate.

15. Internal olefin sulphonates as claimed in one or more of the claims 9-14, wherein the sulphonates are (earth) alkali metal sulphonates or ammonium sulphonates.

16. Internal olefin sulphonates as claimed in claim 15, wherein the sulphonates are sodium or potassium sulphonates.

**Patentansprüche**

1. Ein Verfahren zur Herstellung von Sulfonaten von internen Olefinen, das das Umsetzen eines internen Olefins mit 8 bis 26 Kohlenstoffatomen in einem Filmreaktionsgefäß mit einem Sulfonierungsmittel in einem Molverhältnis von Sulfonierungsmittel zu internem Olefin von 1:1 bis 1,25:1 während Kühlens des Reaktionsgefäßes mit einem Kühlmittel mit einer Temperatur von 35 °C nicht übersteigend, unmittelbares Neutralisieren des erhaltenen Reaktionsproduktes der Sulfonierungsstufe, ohne Extrahieren nicht umgesetzten internen Olefins und Hydrolysieren des neutralisierten Reaktionsproduktes umfaßt.

2. Ein Verfahren wie in Anspruch 1 beansprucht, wobei das Kühlmittel Wasser mit einer Temperatur im Bereich von 0 °C bis 25 °C ist.

3. Ein Verfahren wie in den Ansprüchen 1-2 beansprucht, wobei das Sulfonierungsmittel Schwefeltrioxid ist.

4. Ein Verfahren wie in einem oder mehreren der Ansprüche 1-3 beansprucht, wobei das interne Olefin 13 bis 22 Kohlenwasserstoffatome umfaßt.

5. Ein Verfahren wie in einem oder mehreren der Ansprüche 1-4 beansprucht, wobei das Verfahren in einer solchen Weise durchgeführt wird, daß eine Alterungsstufe gänzlich weggelassen wird.

6. Ein Verfahren wie in einem oder mehreren der Ansprüche 1-5 beansprucht, wobei die Umsetzung in einem Rieselfilmreaktionsgefäß durchgeführt wird.

7. Ein Verfahren wie in einem oder mehreren der Ansprüche 1-6 beansprucht, wobei während der Neutralisierung/Hydrolyse das Reaktionsprodukt der Sulfonierungsstufe sofort mit einer wäßrigen Lösung einer Base vermischt wird.

8. Ein Verfahren wie in Anspruch 7 beansprucht, wobei das Vermischen durch Rühren, durch Zugabe eines Co-Lösungsmittels oder durch Zugabe eines Phasenübertragungsmittels bewirkt wird.

9. Sulfonate von internen Olefinen mit 8 bis 26 Kohlenstoffatomen, dadurch gekennzeichnet, daß Sulfonate von internen Olefinen mindestens 10 Gew% beta-Hydroxysulfonate umfassen, bezogen auf das Gewicht der Gesamtsulfonate.

10. Sulfonate von internen Olefinen wie in Anspruch 9 beansprucht, gekennzeichnet durch mindestens 25 Gew% beta-Hydroxysulfonate.

11. Sulfonate von internen Olefinen wie in Anspruch 10 beansprucht, gekennzeichnet durch mindestens 50 Gew% beta-Hydroxysulfonate.

12. Sulfonate von internen Olefinen wie in Anspruch 11 beansprucht, gekennzeichnet durch 50-90 Gew% beta-Hydroxysulfonate.

13. Sulfonate von internen Olefinen wie in Anspruch 12 beansprucht, gekennzeichnet durch 60-85 Gew% beta-Hydroxysulfonate.

14. Sulfonate von internen Olefinen wie in einem oder mehreren der Ansprüche 9-13 beansprucht, dadurch gekennzeichnet, daß diese Sulfonate von internen Olefinen eine Menge von ungesättigten Sulfonaten enthalten.

15. Sulfonate von internen Olefinen wie in einem oder mehreren der Ansprüche 9-14 beansprucht, wobei die Sulfonate Alkalimetall-, Erdalkalimetall- oder Ammoniumsulfonate sind.

16. Sulfonate von internen Olefinen wie in Anspruch 15 beansprucht, wobei die Sulfonate Natrium- oder Kaliumsulfonate sind.

## Revendications

1. Un procédé pour la préparation de sulfonates d'oléfines internes, selon lequel on fait réagir dans un réacteur à couche mince une oléfine interne ayant de 8 à 26 atomes de carbone avec un agent de sulfonation, dans un rapport molaire de l'agent de sulfonation à l'oléfine interne compris entre 1:1 et 1,25:1 tout en refroidissant le réacteur avec un agent de refroidissement ayant une température ne dépassant pas 35 °C, on neutralise directement le produit de réaction de l'étape de sulfonation obtenu et, sans extraire l'oléfine interne n'ayant pas réagi, on hydrolyse le produit de réaction neutralisé.

2. Un procédé selon la revendication 1, dans lequel l'agent de refroidissement est de l'eau ayant une température comprise entre 0 °C et 25 °C.

**3.** Un procédé selon les revendications 1-2, dans lequel l'agent de sulfonation est de l'anhydride sulfurique.

**4.** Un procédé selon une ou plusieurs des revendications 1-3, dans lequel l'oléfine interne comprend de 13 à 22 atomes de carbone.

**5.** Un procédé selon une ou plusieurs des revendications 1-4, qui est mis en oeuvre de manière que toute étape de vieillissement soit omise.

**6.** Un procédé selon une ou plusieurs des revendications 1-5, dans lequel la réaction est conduite dans un réacteur à film ruisselant.

**7.** Un procédé selon une ou plusieurs des revendications 1-6, dans lequel durant la neutralisation/hydrolyse, le produit de réaction de l'étape de sulfonation est mélangé intimement avec une solution aqueuse d'une base.

**8.** Un procédé selon la revendication 7, dans lequel l'opération de mélange est effectuée par agitation, par l'addition d'un cosolvant ou par l'addition d'un agent de transfert de phase.

**9.** Des sulfonates d'oléfines internes ayant de 8 à 26 atomes de carbone caractérisés en ce que ces sulfonates d'oléfines internes comprennent au moins 10 % en poids de bêta-hydroxy sulfonates, par rapport au poids total des sulfonates.

**10.** Des sulfonates d'oléfines internes selon la revendication 9, caractérisés par au moins 25 % en poids de bêta-hydroxy sulfonates.

**11.** Des sulfonates d'oléfines internes selon la revendication 10, caractérisés par au moins 50 % en poids de bêta-hydroxy sulfonates.

**12.** Des sulfonates d'oléfines internes selon la revendication 11, caractérisés par 50 à 90 % en poids de bêta-hydroxy sulfonates.

**13.** Des sulfonates d'oléfines internes selon la revendication 12, caractérisés par 60 à 85 % en poids de bêta-hydroxy sulfonates.

**14.** Des sulfonates d'oléfines internes selon une ou plusieurs des revendications 9-13, caractérisés en ce que ces sulfonates d'oléfines internes contiennent une certaine quantité de sulfonate insaturé.

**15.** Des sulfonates d'oléfines internes selon une ou plusieurs des revendications 9-14, dans lesquels les sulfonates sont des sulfonates de métaux alcalins ou alcalino-terreux ou des sulfonates d'ammonium.

**16.** Des sulfonates d'oléfines internes selon la revendication 15, dans lesquels les sulfonates sont des sulfonates de sodium ou de potassium.